Europäisches Patentamt

European Patent Office

Office européen des brevets

(11) Publication number: **0 457 450 A1**

# EUROPEAN PATENT APPLICATION

(21) Application number: **91303742.0**

(22) Date of filing: **25.04.91**

(51) Int. Cl.5: **G01N 33/36**

(30) Priority: **15.05.90 GB 9010862**

(43) Date of publication of application:
**21.11.91 Bulletin 91/47**

(84) Designated Contracting States:
**CH DE FR IT LI**

(71) Applicant: **RIETER SCRAGG LIMITED**
**Langley**
**Macclesfield Cheshire, SK11 0DF(GB)**

(72) Inventor: **Eaton, David Charles**
**66 Brown Edge Road**
**Buxton, Derbyshire(GB)**
Inventor: **Brookes, Glenn**
**7 Adlington Road**
**Bollington, Cheshire(GB)**

(74) Representative: **Graves, Ronald**
**Rieter Scragg Limited Langley**
**Macclesfield Cheshire, SK11 0DF(GB)**

(54) **Yarn quality grading method.**

(57) A method of on-line quality grading a package (21) of textured yarn (24) simultaneously with the texturing of the yarn and winding it onto the package (21) comprises measuring at successive short first time intervals a parameter of the textured yarn, eg its velocity, calculating averages of the parameter (Vav) over successive longer second time intervals, noting any averages (Vav) which deviate from a predetermined limit range, and calculating a quality fault level indicative of the quality grading of the package (21) from the cumulative magnitudes of the noted deviations. A transient fault level in respect of a short term transient fault is calculated from the sum of the excess parameter values above a predetermined transient fault level and the duration of the fault. A quality fault level in respect of longer duration deviations is calculated by algorithmic or frequency distribution methods, the transient fault levels and the quality fault levels being compared with at least one predetermined threshold value to give the quality grading of the yarn package (21).

FIG. 1

This invention relates to a quality grading method in relation to textured yarn, and in particular to such grading following the on-line monitoring of textured yarn during the texturing process.

Textured yarn quality has traditionally been monitored off-line, i.e. after texturing has been completed, by testing a sample of yarn taken from the end of a finished package. Typical test methods include knit-and-dye, bulk measurement and, more recently, TYT (textured yarn testing), all of which are destructive of the sample tested. For certain critical end uses all packages produced might be tested, but usually only a sample of the total package production is selected for testing. This testing method has the drawback that it interrupts the steady flow of packages from the texturing process, which is particularly serious in automated plants. Also intermittent or periodic faults within a package may go completed undetected.

To overcome the abovementioned disadvantages the textured yarn quality may be continuously monitored on-line, by continuously measuring the crimp velocity as described in British Patent Application No 2200756. However such a method produces thousands of crimp velocity values in relation to the textured yarn on a single package, and all of such data must be studied in order to obtain a single quality grading for that package as a whole. This is also time consuming and would prevent the steady flow of quality graded packages from the texturing process.

It is also known to continuously monitor yarn tension values during the texturing process, and to count both the number of tension deviations outside a predetermined floating limits range which encompasses the short-term average tensions, and the number of tension deviations outside a predetermined fixed global limits range, which occur during the production of a package of textured yarn. However the floating limits must be pre-set before commencing the texturing of the yarn for each particular yarn type and process requirement, and this is a difficult and time consuming procedure.

It is an object of the present invention to provide a method of quality grading a package of textured yarn which avoids or minimises the abovementioned disadvantages of the prior known methods.

The invention provides a method of quality grading a package of textured yarn simultaneously with texturing the yarn and winding the textured yarn onto the package, comprising measuring a parameter of the textured yarn at successive first time intervals, calculating an average of the parameter during each of successive second time intervals which are greater than the first time intervals, noting any average parameter values which deviate outside a predetermined limit range of the parameter, and calculating a quality fault level indicative of the quality grading of the package from the cumulative magnitudes of the noted deviations.

The parameter measurement may be the measurement of the velocity of the textured yarn, and the measurement may be taken immediately prior to winding the textured yarn onto the package.

The first time interval may be less than 0.2 secs, and may be in the range 50 to 150 milliseconds, for example 96 millisecs.

The second time interval may be in the range 0.5 secs to 5 secs, and may be 0.96 secs, whereby 10 parameter measurements taken at intervals of 96 millisecs are averaged to give a transient average parameter value in respect of each successive second time interval. The quality fault level calculation may comprise determining, in respect of each noted deviation, the integral with respect to time, for the duration of the noted deviation, of the excess of the parameter measurements above a predetermined transient limit. The calculation may also comprise expressing the integral as a proportion of the product of the parameter of untextured yarn and the duration of the noted deviation, to give a transient fault level of the noted deviation. The untextured yarn parameter may be the velocity of the yarn as it is wound on the package. The cumulative transient fault level of the noted deviations may be compared with one or more predetermined threshold values at successive third time intervals, which may be between 30 secs and 2 mins, for example 1 minute, to give a quality grading in respect of the yarn package.

Alternatively the second time interval may be in the extended range 30 secs to 2 mins, for example 1 minute, whereby 625 parameter measurements taken at intervals of 96 millisecs are averaged to give an extended average parameter value in respect of each successive extended second time interval. The quality fault level (QFL) calculation may then comprise a calculation in accordance with the algorithms

QFL = (FMmax + FMmean)/2 and

$$FM = 100 \left[ \frac{VYarn - VLimit}{VF - Vlimit} \right] \quad where$$

FM max and FM mean are maximum and mean values respectively during a package build of the fault magnitude calculated according to the second algorithm above.

**VYarn** is a textured yarn velocity measurement (m/min)

**VLimit** is predetermined package limit parameter value (m/min) and

**VF** is the output feed shaft surface velocity (m/min).

Alternatively a more statistical reduction of the data may be made in order to determine an overall QFL. The alternative method comprises determining a frequency distribution of extended average parameter values relative to a predetermined package limit, and calculating a quality fault level from modal and limit values of the extended average parameter values of the frequency distribution. In this case the quality fault level (QFL) may be calculated from the algorithm :

$$QFL \quad = \quad \frac{(Valarm \ x \ Vmode) \ + \ K_1 Vmax}{Vlimit} \quad +$$

$$\frac{(K_2 CValarm \ x \ CVmode) \ + \ K_3 CVmax}{CVlimit} \quad + \quad \frac{100AS}{Ld}$$

where Valarm = the number of extended average parameter values, $\overline{Vav}$, in excess of the package limit Vlimit as a percentage of the number of extended second time intervals during standard package build. (m/min)

Vmode = the modal value relative to Vlimit of the extended average parameter value frequency distribution. (m/min)

Vmax = the maximum value relative to Vlimit of the extended average parameter values. (m/min)

CValarm = the number of coefficients of variation, of the parameter measurements taken during an extended second time interval, in excess of the package limit coefficient of variation CVlimit as a percentage of the number of extended second time intervals during a standard package build.

CVmode = one tenth of the modal value relative to CVlimit of the coefficients of variation frequency distribution.

CVmax = the maximum coefficient of variation.

CVlimit = a predetermined package limit coefficient of variation.

AS = a multiplier taking the value 1 if any alarm violation ($\overline{Vav}$ in excess of Vlimit) has occurred, and zero if not.

Ld = the number of extended second time intervals during standard package build, and $K_1$, $K_2$ and $K_3$ are constants which empirically determined are approximately 20, 10 and 20 respectively.

The quality fault level may be compared with one or more predetermined threshold values at the successive third time intervals, to give a quality grading in respect of the yarn package.

The method may comprise simultaneously calculating a cumulative transient fault level and a quality fault level at the successive third time intervals and comparing the levels with one or more respective threshold values to give a quality grading in respect of the yarn package.

The invention will now be described with reference to the accompanying drawings in which :

**Fig 1** is a schematic diagram showing a yarn texturing machine on which the method of the present invention may be performed.

**Fig 2** is a typical graph of yarn speed, Vyarn, against time derived from yarn velocity measurements taken every 96ms, during two minutes of yarn processing.

**Fig 3** is a graph, corresponding with the measurements shown in Fig 2, of average velocity values, Vav, evaluated at successive time intervals of approximately 1 sec.

**Fig 4** is a part of the graph of Fig 2 to an enlarged scale.

**Fig 5** is a graph of yarn speed, Vyarn, against time derived from yarn velocity measurements taken over an extended period of time during which a change of supply package occurred.

**Fig 6** is a graph of average velocity values, $\overline{Vav}$, evaluated at successive time intervals of 1 min showing a typical steady state or extended fault.

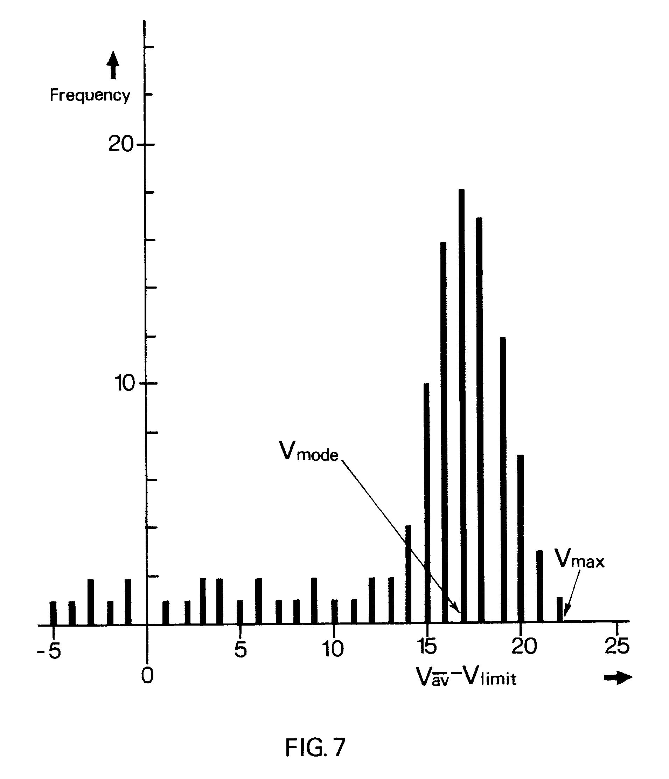

**Fig 7** shows a frequency distribution based on the results shown in Fig 5.

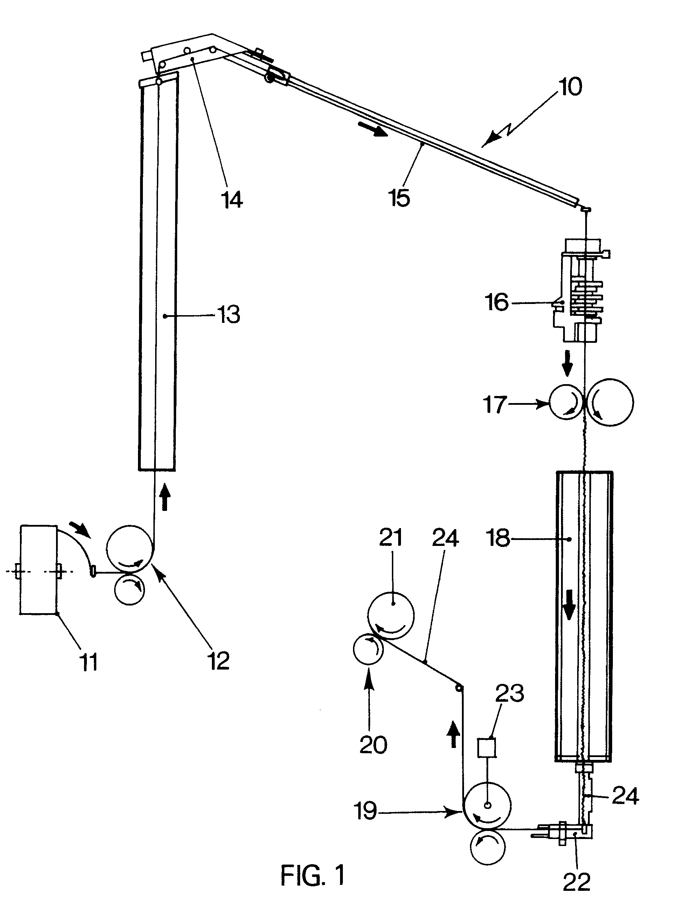

Referring now to Fig 1 there is shown diagrammatically one yarn position of a false twist texturing machine 10 comprising a creel (not shown) in which a yarn supply bobbin 11 is mounted, a first feed means 12 in the form of a pair of nip rollers, a primary heater 13, a turnround guide sledge 14, a cooling track 15, a false twist device 16, an intermediate feed/drawing means 17, also comprising a pair of nip rollers, a secondary heater 18, a take-up feed means 19 also comprising a pair of nip rollers, and a package winding means 20 for winding a package 21 of textured yarn. In the case of single heater machines the intermediate feed means 17 and the secondary heater 18 are omitted. Also provided in the apparatus shown in Fig 1 is a yarn velocity measuring transducer 22 and a roller speed measuring device 23. The velocity measuring transducer 22 measures the velocity Vyarn of the textured yarn 24 immediately prior to it being fed by the take-up feed means 19 to the package winding means 20, and the roller speed measuring device 23 provides a signal proportional to the surface speed $V_F$ of the take-up feed means 19 and hence the speed of the yarn 24 as it passes in contact with those surfaces. The difference between the speeds measured by the device 23 and the transducer 22 will be proportional to the amount of crimp in the textured yarn 24, being zero in the case of untextured or flat yarn.

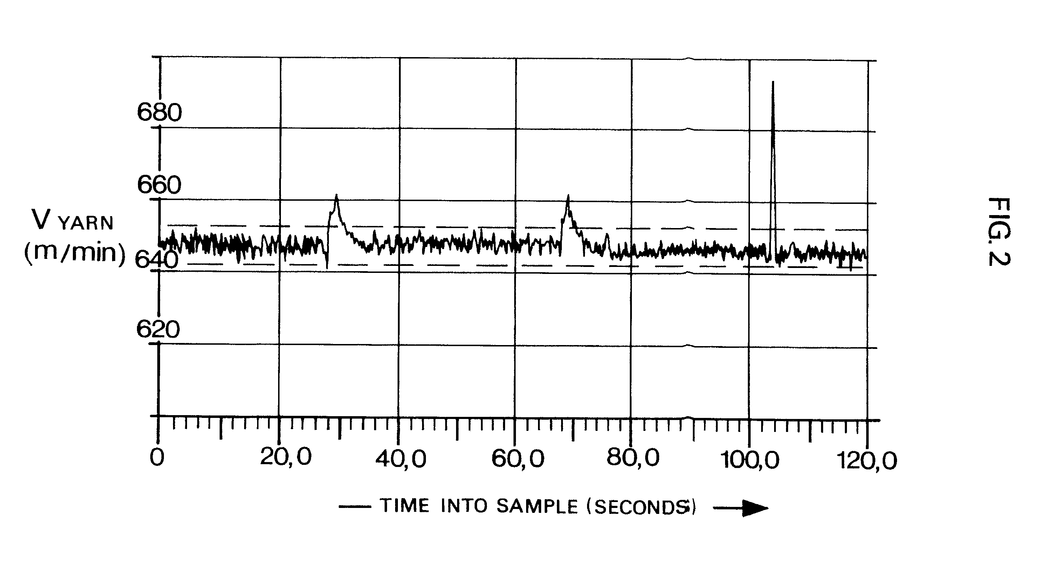

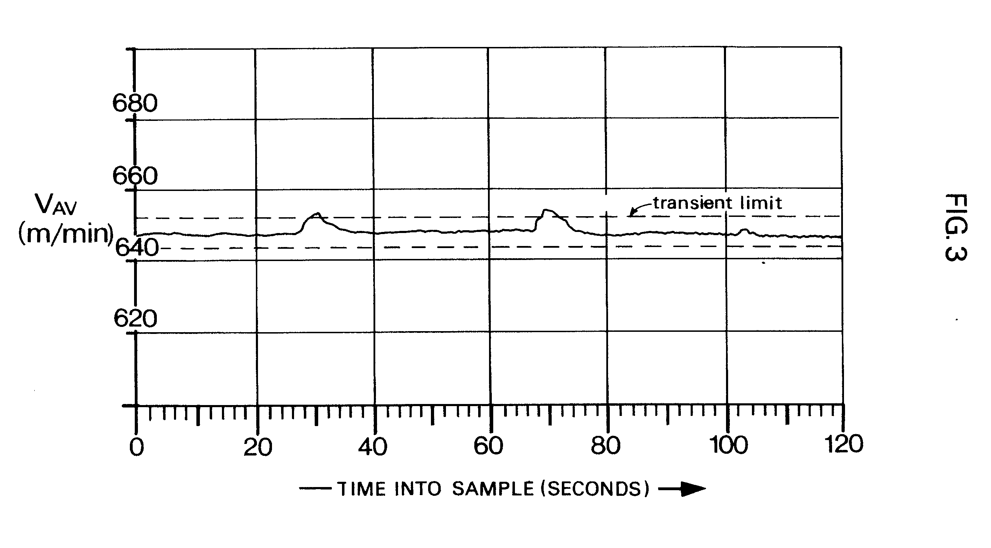

Typical velocity readings taken from the transducer 22 during texturing and winding up of the yarn 24 are shown in Fig 2, which represents such velocity readings taken over a total time of 2 minutes. The transducer 22 is more fully described in British Patent Application No 2200756, and a velocity reading Vyarn is obtained every 96 milliseconds. This first time period is convenient having regard to the sampling frequency and the capabilities and economics of the equipment, but other time periods may be used if preferred. The results shown in Fig 2 illustrate two transient disturbances in the yarn processing, such as might be caused by yarn knots, occurring at approximately 24 and 64 seconds into the yarn sample, and one transient "spike" at approximately 100 seconds. In the case of the first two transients, there is a rapid increase in yarn velocity Vyarn, equivalent to a reduction of crimp in the yarn 24, lasting for about 1 second followed by a gradual return to the normal running conditions. The normal running or "background" conditions show many small velocity variations consequent upon the modern high speed draw-texturing process being dependent upon frictional interactions between the yarn 24 and the various parts of the machine 10 with which it comes into contact, in particular the discs of the friction twisting unit 16. These small variations do not however generally affect the yarn quality, but the transients illustrated may be such as to cause significant faults in the processed yarn. Such transients are caused by short lived disturbances which create a temporary loss of control in the texturing zone, resulting in short lengths of untextured or partially textured yarn. The difference between a transient which is likely to cause a significant fault in the yarn and one which is not, is a matter of both the magnitude of the deviation from the background level and the duration of such deviation. In consequence it can be seen from Fig 3 that the first two transients are significant from the fault point of view but the "spike" transient is not. Fig 3 is a graph showing average velocities Vav over second time intervals of 0.96 secs (ie 10 velocity measurements). Such averages have the effect of smoothing out the background and the "spike" transient, which did not last long enough to have caused a loss of process control. However the other two transients are still clearly discernable, and are noted as such since the maximum average velocities associated with these transients exceeds the predetermined transient limit shown in Fig 3.

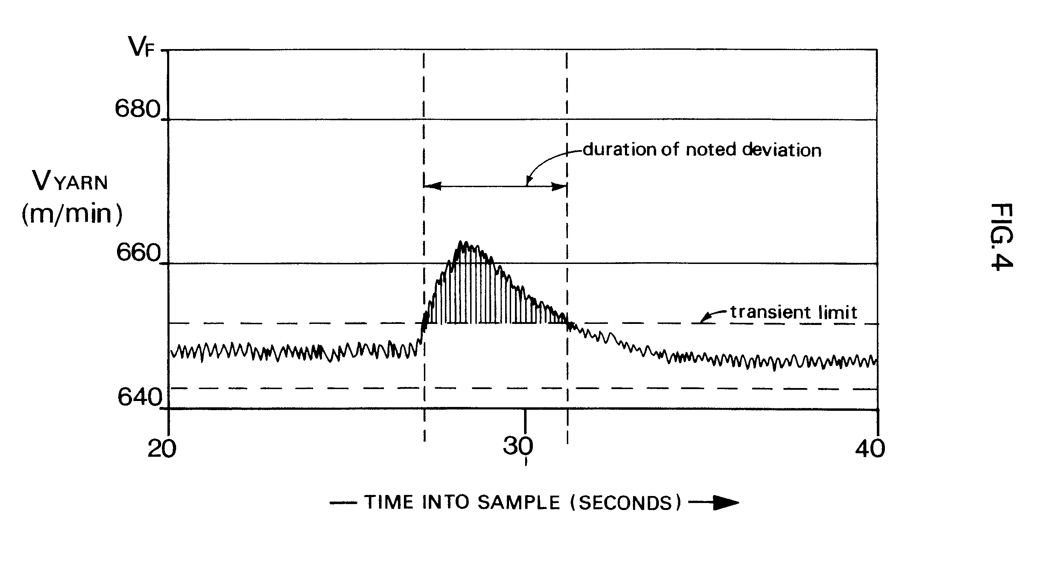

Referring now to Fig 4 there is shown a part of Fig 2, to an enlarged scale, which encompasses one of the noted transients determined from an average velocity deviation beyond the transient limit as described above. In this case, part of the graph lies above the transient limit, and the shaded area represents the integral with respect to time for the duration of the noted deviation of the excess of the velocity measurements above the transient limit. This area is representative of the magnitude of the transient having regard to both its maximum value and its duration. In order to obtain a figure which is representative of the magnitude of the fault likely to be produced in the textured yarn, the integral is expressed as a percentage of the worst possible value such integral would have if the yarn velocity were to equal the surface speed $V_F$ of the feed means 19, ie a situation in which there would be no crimp in the yarn due to a complete collapse of the texturing process. This worst case integral equals the product of the difference between $V_F$ and the transient limit and the duration of the noted transient. As previously mentioned the roller speed measuring device 23 measures the speed $V_F$ so that the "worst case" integral or transient magnitude is readily calculable. The Transient Fault Level (TFL) is then the calculated transient magnitude (ie the shaded area in Fig 4) expressed as a percentage of the "worst case" transient magnitude calculated as specified above.

The accumulated TFL is compared every third time interval, for example every minute, with predetermined transient threshold levels so as to automatically grade the growing yarn package with respect to its transient quality. The transient threshold levels are determined by the yarn user having regard to the particular end use of the yarn and the quality of textured yarn required for such end use.

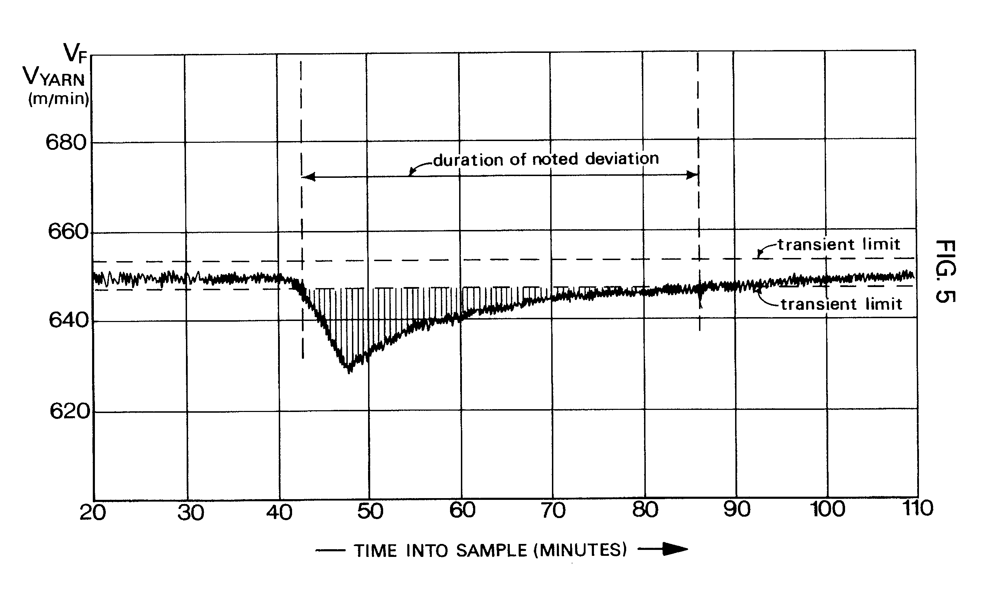

In addition to transients as described above there are also steady state, or extended, disturbances. These do not disrupt process equilibrium but shift process equilibrium conditions to a new level. Such extended disturbances may last for a minute or for several hours. To monitor such disturbances the velocity measurements Vyarn described above are averaged over much longer second time intervals than specified in relation to transient monitoring. In this case average velocities $\overline{Vav}$ are calculated over second time intervals of 1 minute (ie 625 velocity measurements), and a graph of such averages will determine when a deviation has occurred, as previously described in relation to Fig 3 for short duration deviations. Referring now to Fig 5 there is shown a graph of yarn speed, Vyarn against time derived from yarn velocity measurements taken over an extended period of 90 minutes. During such time, the velocity readings shown are characteristic of those experienced on the occasion of the changeover from one supply package to another. It is believed that the spin finish oil tends to be absorbed by the paper tube from the inner layers of a package and to be evaporated from the outer layers, leading to relatively dry yarn at the end of one supply package and at the start of a new supply package. This is believed to dry the surface of the friction false twisting discs, leading to greater twist levels and yarn bulk. The decrease in yarn velocity can take about 5 minutes and the increase back to the original normal running value can take up to one hour. As with the case shown in Fig 4, the Fault level can be caluculated as the Fault Magnitude (ie the shaded area in Fig 5) expressed as a percentage of the "worst case" Fault Magnitude ($V_F$ - transient limit x duration of noted deviation). However, since for 1 minute averaged data each averaged yarn velocity $\overline{Vav}$ represents several hundred metres of yarn, a steady state Fault Magnitude may be calculated for each $\overline{Vav}$ value as follows

$$FM = \left[ \frac{\overline{Vav} - VLimit}{V_F - VLimit} \right] \times 100$$

A simple measure of the cumulative effect of a fault such as that shown in Fig 5 is given by the algorithm, updated each minute to form a running Quality Fault Level,

QFL = [FM max + FM mean] /2

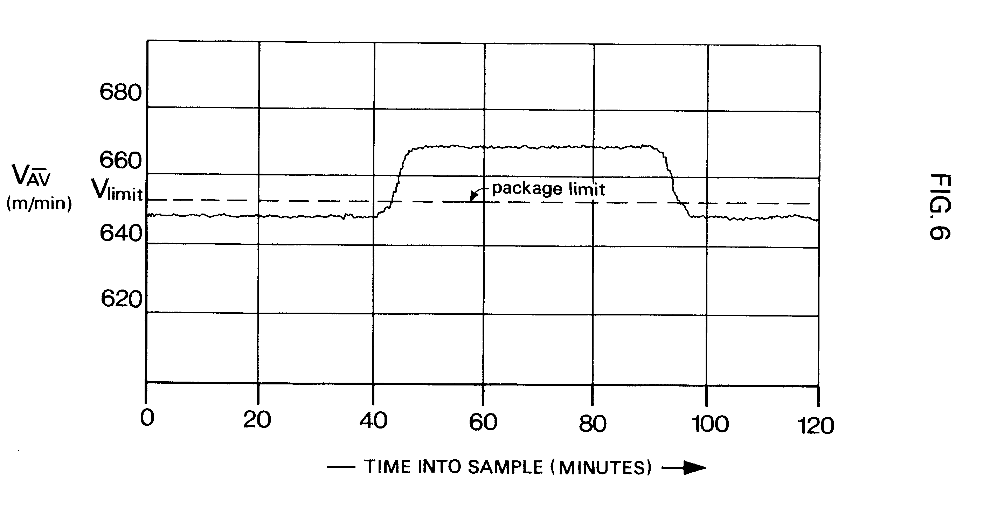

As an alternative approach to the calculation of fault level for such extended disturbances, reference may be had to the graph of average velocities over second time intervals of 1 minute as described above, and Fig 6 shows a typical graph of such averages in the case of an extended disturbance lasting from about 40 mins into the sample to about 92 mins. From these results a frequency distribution as shown in Fig 6 is produced and Vmode, Vmax and AS determined. At the same time the coefficient of variation of the velocity measurements is determined at each interval, and by the same averaging procedure a frequency distribution of the coefficients of variation is produced to establish CValarm limit violations. The addition of the coefficient of variation fault data as a factor in the QFL calculation, in accordance with the previously specified formula, is to take account of any uniformly variable process fault which would otherwise go undetected since the averaging process would reduce the velocity readings to the background average and there would be no effect on the frequency distribution shown in Fig 6. The results also enable Valarm, CValarm, CVmode and CVmax to be calculated and hence the Quality Fault level QFL. The calculation is updated every third time interval, for example every minute, and compared with predetermined threshold values in order to grade the package as it is wound. All of the calculations are performed by feeding the signals from the transducer 22 and roller speed measuring device 23 to a suitable programmed computing device (not shown).

The transient and package limits which are applied to Vav, $\overline{Vav}$ and CV respectively are calculated whenever a process specification for texturing a particular yarn type is set up, and such limits are stored in the computing device so that they are immediately available whenever that process specification is performed again. The limits are closely toleranced, by statistically assessing the background variation of several, typically 12, normally running packages over a sufficient period to establish the magnitude of such variations, and centred individually on the initial mean yarn velocity for each position of a texturing machine during the first five minutes of each package wind.

By means of the invention each package of textured yarn being produced on a texturing machine is continuously monitored for yarn quality as regards both short term transients and extended process deviations, and the packages are continuously assessed against predetermined quality threshold levels to give a current quality grade for each package as it is built.

## Claims

1. A method of quality grading a package of textured yarn comprising texturing the yarn, measuring a parameter of the textured yarn at successive first time intervals, and winding the textured yarn (24) onto the package (21) characterised by simultaneously calculating an average of the parameter (Vav) during each of successive second time intervals which are greater than the first time intervals, noting any average parameter values (Vav) which deviate outside a predetermined limit range of the parameter, and calculating a quality fault level indicative of the quality grading of the package (21) from the cumulative magnitudes of the noted deviations.

2. A method according to claim 1 characterised in that the parameter measurement is the measurement of the velocity (VYARN) if the textured yarn (24) taken immediately prior to winding the textured yarn (24) onto the package (21).

3. A method according to claim 1 or claim 2 characterised in that the first time interval is in the range 50 to 150 milliseconds, and the second time interval is in the range 0.5 seconds to 5 seconds.

4. A method according to claim 3 characterised in that the calculation comprises determining in respect of each noted deviation, the integral with respect to time, for the duration of the noted deviation, of the excess of the parameter measurements (VYARN) above a predetermined transient limit, and determining a transient fault level of each noted deviation which is the inte gral expressed as a proportion of the product of the parameter of untextured yarn (VF) and the duration of the noted deviation.

5. A method according to claim 4 characterised in that the calculation comprises determining a cumulative transient fault level of the noted deviations and comparing the cumulative transient fault level with at least one predetermined threshold value at successive third time

6. A method according to claim 1 or claim 2 characterised in that the first time interval is in the range 50 to 150 milliseconds, and the second time interval is an extended interval in the range 30 seconds to 2 minutes.

7. A method according to claim 6 characterised in that the calculation comprises calculating a Quality Fault Level in accordance with the algorithms QFL = (FMmax + FMmean)/2 and

$$FM = 100 \left[ \frac{Vyarn - Vlimit}{VF - Vlimit} \right], \text{ and in that}$$

the terms of the algorithms are as herein defined.

8. A method according to claim 6 characterised in that the calculation comprises determining a frequency distribution of extended average parameter values (Vav) relative to a predetermined package limit, and calculating a Quality Fault Level from modal (Vmode) and limit (Vlimit) values of the extended average parameter ($\overline{Vav}$) values of the frequency distribution.

9. A method according to claim 8 characterised in that the calculation comprises calculating a Quality Fault Level in accordance with the algorithm

$$QFL = \frac{(Valarm \times Vmode) + K_1 Vmax}{Vlimit} + \frac{(K_2 CValarm \times CVmode) + K_3 CVmax + 100AS}{CVlimit \quad Ld}$$

and in that the terms of the algorithm are as herein defined.

10. A method according to claim 7 or claim 9 characterised in that the calculation comprises comparing the Quality Fault Level with at least one predetermined threshold value at successive third time intervals to give a quality grading in respect of the yarn package (21).

FIG. 1

## FIG. 2

# FIG. 3

## FIG. 4

# FIG. 5

# FIG. 6

FIG. 7

# EUROPEAN SEARCH REPORT

EP 91 30 3742

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | RESEARCH DISCLOSURE, no. 120, April 1974, pages 48-50, disclosure no. 12029; "Crimp characterization instrument" <br> * Page 48, right-hand column, paragraphs 1-2,5; page 49, left-hand column, paragraph 1 - right-hand cclumn, paragraph 2; page 53, left-hand column, paragraph 3 - right-hand column, paragraph 2; figures 1-3 * <br> — — — | 1,2 | G 01 N 33/36 |
| A | WO-A-8 302 665 (LAWSON-HEMPHILL, INC.) <br> * Page 4, line 14 - page 5, line 18; page 8, line 5 - page 10, line 20; abstract; figures 1-3 * <br> — — — | 1,2 | |
| D,A | EP-A-0 271 252 (RIETER SCRAGG LTD) <br> * Page 4, line 1 - page 5, line 12; abstract; figure 1 * <br> — — — — — | 1,2 | |

| | |
|---|---|
| | TECHNICAL FIELDS SEARCHED (Int. Cl.5) |
| | G 01 N |

The present search report has been drawn up for all claims

| Place of search | Date of completion of search | Examiner |
|---|---|---|
| The Hague | 02 September 91 | BOSMA R.A.P. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document